# EUROPEAN PATENT APPLICATION

(11) **EP 2 671 545 A2**
(43) Date of publication of application: **11.12.2013**
(21) Application number: 12461544.4
(22) Date of filing: 11.09.2012
(51) Int. Cl.: A61F 5/01

(54) **Method for producing orthopaedic appliances, specifically those provided with rigid components for active stimulation of the injured area**

(30) Priority: 04.06.2012 PL 39940812
(71) Applicant: MTB Poland sp. z o.o., 95-070 Aleksandrow (PL)
(72) Inventor: Piotrowski, Andrzej, 91-496 Lódz (PL); Herka, Katarzyna, 98-105 Kwiatkowice las (PL)
(74) Representative: Rumpel, Alicja

(57) **Abstract**

The method for producing orthopaedic appliances, specifically those provided with rigid components for active stimulation of the injured area, according to this invention is comprised of the following steps:
• cutting of the textile materials on a plotter,
• moulding of the composite,
• processing of the composite,
• finishing of the composite surface,
• producing of textiles for the body of the orthopaedic appliance,
• stabilising of the knitted fabric,
• welding of the textile materials and composites,
• sewing to finish.
wherein the textile materials to be cut on the plotter are selected from among natural textile materials fortified with an admixture of fibres with low melting point.

## Description

The invention relates to the method for producing orthopaedic appliances, specifically orthopaedic appliances provided with rigid components for active stimulation of the injured area, specifically orthopaedic appliances provided with an electric/electronic rehabilitation and/or diagnostic system,

Orthopaedic orthoses and elements for bracing medical equipment used as orthopaedic appliances for limbs and joints used to be made of steel, mostly surgical steel. Given the substantial weight of the devices made in this manner, they began to be replaced with elements made of plastic, and currently also of composite materials.

There is known a method for producing orthopaedic insoles according to the patent description no. PL199255, wherein the layers of orthopaedic insoles, composed of at least three layers, have thermoplastic properties. After the layers are put together, the material of the insole is pressed, while heating the press ram. As a result the layers are bonded together and the ready-to-use product is not susceptible to delamination.

There are also known methods for producing composite elements provided with electric conductivity properties, wherein at least one layer of the composite conducts electrical voltage applied thereto or dissipates electrostatic charge accumulated on the surface of the composite as a result of interactions with the environment. Such solutions are known for example from the description of the invention no. US20090269530 which refers to the method for manufacturing containers (storage tanks) for dangerous liquids. This description of the invention disclosed a container for storing flammable substances, composed of at least three layers, where the internal layer is made of the original material and the adjacent layer dissipates electric charges. The layer that dissipates electric charges is made of plastic doped with e.g. graphite and closed on the outside with a layer of nonconducting plastic.

Also the bracing elements of the known solutions in the area of orthopaedic appliances are made as composite elements. However, in the case of such medical applications the path of electrical impulses carried through the conducting elements to the patient's body must be precisely defined. Therefore, it is not possible to use any known composite materials which conduct electrical voltage with their entire surface or a layer thereof to stimulate the selected single muscle groups as the application of such solutions would cause multiple shock induced by an electrical impulse which could affect the rehabilitation process or the patient's health.

More and more often textile materials, designed for monitoring the health condition of people wearing clothes made of such materials, are used for diagnosing or treating diseases in which no immobilisation or bracing of the patient's body is required. The conducting, measuring, bracing and electrostatic charge dissipating elements - known and widely used in electronics - are worked into the structure of the material without changing the essential dimensions and weight of the device. Depending on the intended use of the clothing, it is provided with chemical, optical, mechanical, electrical, thermal, magnetic or acoustic sensors. Sensors are generally made of optical fibres connected with measuring heads, tunnel sensors (Softswitch Technology), Gorix, ElekTex. Active elements are also used, especially in military clothing, e.g. to stop bleeding, such as: electroactive polymers, dielectric elastomers, carbon nanotubes which change thermal energy into mechanical energy or heaters (conducting fibres).

The potential of textronics proved to be useful to the extent that today there are international projects under implementation, such as e.g. the Wealthy project which aims at creating a system for monitoring a user's health condition with the use of a range of intelligent sensors and an interface as well as based on modern data processing and transmitting methods, which could signal for medical assistance in the event of cardiac arrest or loss of consciousness.

Clothing with active elements built into the structure of the textile material is also used as an instrument for rehabilitation in the form of pressure or electrical stimulation of the muscles.

However, the materials known in the textile industry cannot be applied in orthopaedic appliances in a simple manner when partial or total immobilisation of the injured area is necessary as these materials exhibit significant flexibility, thus preventing appropriate rehabilitation of the injured area.

On the other hand, it is possible to produce a composite brace using textronic material and work it into an orthopaedic appliance, however, that significantly increases the costs of the end product and requires complicated operations to secure sensors built into textiles prior to the application of adhesive thereto which deprives the end product of the required functionality.

Hence, it has become appropriate to develop a method for producing rigid components for orthopaedic appliances provided with instruments for muscular stimulation, specifically bracing elements for orthoses, where such method would not increase the costs of production of the end products and at the same time would ensure appropriate stability and rigidity of such products. An additional object of the invention was to develop such method that would allow eliminating the need to use complicated measures and procedures for protecting sensors placed in the bracing elements of orthopaedic appliances prior to their damage with the applied adhesive.

The method for producing orthopaedic appliances, specifically those provided with rigid components for active stimulation of the injured area, according to this invention offers such opportunity and comprises the following steps:
- cutting of the textile materials on a plotter,
- moulding of the composite,
- processing of the composite,
- finishing of the surface of the composite,
- producing of textiles for the body of the orthopaedic appliances,
- stabilising of the textiles,
- welding of the textile materials and composites,
- sewing to finish.

The textile materials to be cut on the plotter are selected from among natural textile materials, preferably made of flax, jute, cocoa or bamboo fibres, and the natural fibres are fortified with an admixture of fibres with low melting point, preferably polypropylene (PP) and/or polyactic acid (PLA).

The raw material is preferably composed of technical textiles made of natural fibres (preferably flax - Flax Fabric) which contain up to 40% of PLA and/or PP fibres that are plasticised (become soft) under temperature (90-200°C) and slightly higher pressure (1-9 bars), and as a polymer harden the other components of the textile

The composite is preferably moulded with the use of a press, vacuum chamber or RTM device and at least one outer layer of the composite is a medium with at least one electric circuit/electronic system. The medium of the electric circuit/electronic system is preferably foil or another plastic which becomes plasticised under temperature and/or pressure, provided in the form of a film with a circuit/system printed or applied thereon with terminals protected preferably with stickers. The medium of the electronic circuit/system can also preferably be a grooved structure supporting the electric circuit/electronic system made on the surface of the composite.

If the composite is moulded with the use of a press, the medium together with the electric circuit is placed into the mould, and further layers of the composite are put on it. The press is provided with a profile / mould of appropriate shape, heating elements and adjustable clamping force.

If the composite is moulded with the use of a press, it is also possible that the composite component is provided with elements supporting the electric circuit / electronic system, preferably in the form of a grooved structure, with which the electric circuit / electronic system can be connected after it is taken off the press.

After all layers are positioned, the press tool presses them to the mould and the layers of the fabric are combined under the temperature of 90-200°C and pressure of 9 bars at maximum into a uniform composite with the shape of the mould.

If the composite is moulded in a vacuum chamber, the medium of the electric circuit / electronic system is put on a mould placed in the chamber and further layers of the composite are put on it. Then the chamber is closed with a vacuum bag and sealed. After the pump is switched on, infusion is initiated which creates subpressure between the "vacuum bag" and the mould. At the same time liquid resin is pressed into the mould and it penetrates any free space in the textile material and permeates it to the electric circuit medium, thus bonding the medium together with the composite. Preferably the subpressure created in the vacuum chamber ranges from 0.3 to 0.9 bars.

The application of the vacuum method allows reducing the content of resin to as much as 50% compared to composites manufactured using traditional methods, with the same or even greater strength of the composite produced in this manner. During the infusion subpressure is generated and as a result the layers of fabrics are compressed with significant reduction of their volume and at the same time completely saturated with resin. Another important advantage of the application of the vacuum infusion technology is the elimination of air bubbles which increases the strength and aesthetic quality of the product. The vacuum infusion technology can be used for producing both moulds and ready-to-use components from composites. More importantly, this process makes it possible to significantly reduce the costs of the production process, especially compared to the RTM method.

If the composite is moulded using the RTM (Resin Transfer Moulding) method, the mould (at least a two-piece mould) is first thoroughly cleaned and degreased, and then coated with a separator, preferably wax. The separator is then polished and subsequently a gel-coat is applied together with the electric circuit /electronic system medium. The gel-coat can be applied using any known method, preferably by spray coating. Then the layers of the textile material as cut out on the plotter are put into the mould. The moulds are closely put together and adhesive is pressed inside them. Resin such as polyester resin, vinyl ester resin, epoxy resin or methacrylate resin is used as adhesive.

The adhesive is continued to be pumped inside until it starts pouring through control outlets on the edges of the mould which means that the textile material is completely saturated in the total volume of the mould. Then the mould is opened and the ready-to-use product is taken out and the mould is prepared for the next cycle.

An important advantage of the RTM technology is the possibility of obtaining products with even and smooth surface on both sides which can be painted to class A and also a relatively short duration of the production process. At the same time the resin transfer moulding allows that the thickness of the walls at each point of the adhesive is precisely determined.

When the composite is ready, the edge processing is initiated to eliminate any technological allowances and also to make holes for screws or rivets. If the edges of the composite are too sharp, they are heated, preferably with a hot air gun, and as a result they are spontaneously rounded.

The composite with the surface prepared in this manner is preferably varnished to protect it against mechanical damage and also for aesthetic reasons.

At the same time and regardless of the preparation of the composite elements, a knitted fabric is produced which is used for the body and ancillary elements of the end product. The body of the orthopaedic appliance is preferably produced as a knitted fabric, preferably using a flat knitting machine. This knitted fabric is thermostabilised to prevent shrinking of the knitted fabric elements of the end product in the use phase. The body of the orthopaedic appliance is also preferably an element sewed from ready-to-use textile materials.

Then the composite elements of the orthopaedic appliance are joined with the knitted fabric body and ancillary elements such as fixing and welded straps. Preferably, adhesive is placed between the body and the rigid composite elements. The rigid composite elements are also preferably placed in a "pocket" or fixed with Velcro or other straps, or attached with appropriate adhesive which has no destructive impact on the structure of the composite and the textile materials. After this operation is finished, the product is sewn to finish and the stickers placed on the electric circuit medium to protect the active sites of the electric circuit which act on the patient's tissue are removed.

The elements of the orthopaedic appliance such as tension straps, surfaces with elements for bracing the product (including a composite insert with an electronic circuit) or other components of the end product are spot welded using a welding press. Then the two layers of textile materials are joined permanently after the adhesive between them melts under temperature and the pressure force.

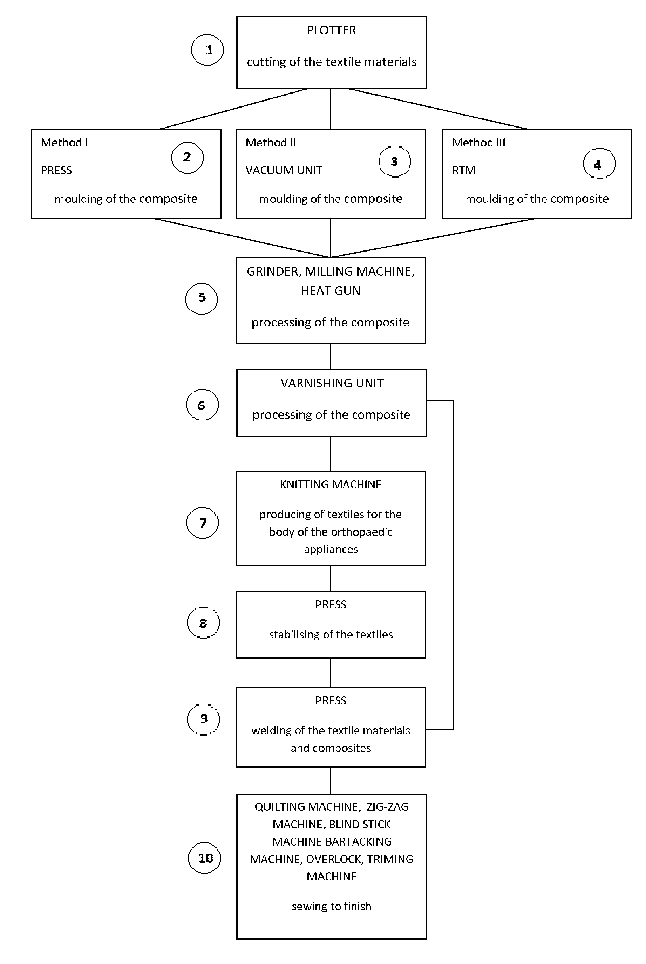

The method for producing orthopaedic appliances, specifically those provided with rigid components for active stimulation of the injured area, is shown schematically in the figure below, where Fig. 1 shows the process flow diagram.

### Example I

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the textiles,
- welding of the textile materials and composites,
- sewing to finish.

And the textile materials to be cut on the plotter are selected from among natural textile materials made of flax, jute, and cocoa or bamboo fibres. The raw material is composed of technical fabrics made of natural fibres which contain up to 40% of PLA and/or PP fibres that are plasticised under temperature (90-200°C) and higher pressure, and as a polymer harden the other components of the fabric.

The composite is moulded with the use of a press, the outer layer of the composite constitutes a medium with an electric circuit/electronic system. The medium of the electric circuit/electronic system is a plastic which is plasticised under temperature and/or pressure, provided in the form of a film with an electronic circuit/system applied thereon with terminals protected with stickers.

When the composite is moulded, the medium together with the electric circuit is placed in the press mould, and further layers of the composite are placed on the medium. The press is provided with a profile / mould of appropriate shape, heating elements and has adjustable pressure force.

After all layers of the composite are placed, the press tool presses them to the mould and the layers of the fabric are combined under the temperature of 90-200°C and pressure of 9 bars at maximum into a uniform composite with the shape of the mould.

When the composite is ready, its edges are processed so as to eliminate any technological allowances and also to make holes for screws or rivets. If the edges of the composite are too sharp, they are heated and as a result they become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. The body of the orthopaedic appliance is produced as a knitted fabric product. This knitted fabric is thermostabilised to prevent shrinking of the knitted fabric elements of the end product in the use phase.

Then the composite elements of the orthopaedic appliance are joined with the knitted fabric body and ancillary elements such as fixing and welded straps. An adhesive is put between the body and the rigid composite elements.

After this operation is finished, the product is sewn to finish and the stickers placed on the medium of the electric circuit to protect the active sites of the electric circuit acting on the patient's tissue are removed.

The elements of the orthopaedic appliance such as tension straps, surfaces with elements for bracing the product (including a composite insert with an electronic circuit) or other components of the end product are spot welded using a welding press. Then the two layers of the textile materials are bonded permanently after the adhesive between them melts under the temperature and the pressure force.

### Example II

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the knitted fabric,
- welding of the textile material and composite,
- sewing to finish.

And the textile materials to be cut on the plotter are selected from among natural textile materials made of flax, jute, and cocoa or bamboo fibres.

The raw material is composed of technical fabrics made of natural fibres which contain up to 40% of PLA and/or PP fibres that are plasticised under temperature (90-200°C) and slightly higher pressure, and as a polymer harden the other components of the fabric.

The composite is moulded with the use of vacuum chamber, the outer layer of the composite is a medium with an electric circuit/electronic system. The medium of the electric circuit/electronic system made of plastic which becomes plasticised under temperature and/or higher pressure, provided in the form of a film with an electronic circuit/system applied to its surface with terminals protected with stickers.

The medium of the electric circuit / electronic system is put on a mould placed in the chamber and further layers of the composite are put on the medium. Then the chamber is closed with a vacuum bag and sealed. After the pump is switched on, infusion is initiated which creates subpressure between the "vacuum bag" and the mould. At the same time liquid resin is pressed into the mould and it penetrates any free space in the textile material and permeates the material to the medium of electric circuit, thus bonding the medium together with the composite. The subpressure created in the vacuum chamber ranges from 0.3 to 0,9 bars.

The application of the vacuum method allows reducing the content of resin to as much as 50% compared to composites manufactured using traditional methods, with the same or even greater strength of the composite produced in this manner. During the infusion process subpressure is generated and as a result the applied fabrics are compressed with significant reduction of their volume and at the same time completely saturated with resin. Another vital advantage of the application of the vacuum infusion technology is the elimination of air bubbles which increases the strength and aesthetic quality of the product.

When the composite is finished, the edges are processed so as to eliminate any technological allowances and also make holes for screws or rivets. If the edges of the composite are too sharp, they are heated and as a result they become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. The body of the orthopaedic appliance is made of knitted fabric which is thermostabilised to prevent shrinking of the knitted fabric elements of the end product in the use phase.

Then the composite elements of the orthopaedic appliance are joined with the knitted fabric body and the ancillary elements such as fixing and welded straps. An adhesive is put between the body and the rigid composite elements.

The elements of the orthopaedic appliance such as tension straps, surfaces with elements for bracing the product (including a composite insert with an electronic circuit) or other components of the end product are spot welded using a welding press. Then the two layers of the textile materials are bonded permanently together after the adhesive between them melts under the temperature and the pressure force.

### Example III

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the knitted fabric,
- welding of the textile materials and composites,
- sewing to finish.

The textile materials to be cut on the plotter are selected from among natural textile materials. The raw material is composed of technical fabrics made of natural fibres which contain up to 40% of PLA and/or PP fibres that are plasticised under temperature and higher pressure, and as a polymer harden the other components of the fabric.

The composite is moulded with the use of a RTM device and the outer layer of the composite is composed of a medium together with an electric circuit/electronic system. The medium of the electric circuit/electronic system is a plastic which is plasticised under temperature and/or pressure, provided in the form of a film with an electronic circuit/system applied on its surface with terminals protected with stickers.

When the composite is moulded, the mould is first thoroughly cleaned and degreased, and then coated with wax. The separator is then polished and next a gel-coat is applied together with an electric circuit / electronic system. Then the layers of the textile material as cut out on the plotter are put into the mould. The moulds are sealed together and an adhesive is pressed inside them. Resin such as polyester resin, vinyl ester resin, epoxy resin or methacrylate resin is used as an adhesive.

The adhesive is continued to be pumped inside until it starts pouring through control outlets on the edges of the mould which means that the textile material is completely saturated in the total volume of the mould. Then the mould is opened and the ready-to-use product is taken out and the mould is prepared for the next cycle.

A great advantage of the RTM technology is the possibility of obtaining products with even and smooth surface on both sides which can be painted to class A, and also a relatively short duration of the production process. At the same time the resin transfer moulding allows that the thickness of the walls is precisely determined at each point of the adhesive.

When the composite is ready, the edges are processed so as to eliminate any technological allowances and to make holes for screws or rivets. If the edges of the composite are too sharp, they are heated and as a result they become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. The body of the orthopaedic appliance is produced as a knitted fabric product. This knitted fabric is thermostabilised to prevent shrinking of the knitted fabric elements of the end product in the use phase.

Then the composite elements of the orthopaedic appliance are joined with the knitted fabric body and the ancillary elements such as fixing and welded straps. An adhesive is put between the body and the rigid composite elements. After this operation is completed, the product is sewn to finish and the stickers placed on the medium of the electric circuit to protect the active sites of the electric circuit acting on the patient's tissue are removed.

The elements of the orthopaedic appliance such as tension straps, surfaces with elements for bracing the product or other components of the end product are spot welded using a welding press. Then the two layers of the textile materials are bonded permanently after the adhesive between them melts under the temperature and the pressure force.

### Example IV

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the knitted fabric,
- welding of the textile materials and composites,
- sewing to finish.

And the textile materials to be cut on the plotter are selected from among natural textile materials made of flax, jute, and cocoa or bamboo fibres. The raw material is composed of technical fabrics made of natural fibres which contain up to 40% of PLA and/or PP fibres that are plasticised under temperature (90-200°C) and higher pressure, and as a polymer harden the other components of the fabric.

The composite is moulded with the use of a press, the outer layer of the composite is composed of a medium of the electric circuit/electronic system in the form of a grooved structure on the surface of the composite, which supports the electric circuit / electronic system.

When the composite is moulded, the medium together with the electric circuit is placed in the press mould, and then further layers of the composite are placed on the medium. The press is provided with a profile / mould of appropriate shape, heating elements and has adjustable pressure force.

After all layers are placed, the press tool presses them to the mould and the layers of the fabric are combined under temperature and pressure into a uniform composite with the shape of the mould.

When the composite is ready, the edges are processed so as to eliminate any technological allowances and also to make holes for screws or rivets. If the edges of the composite are too sharp, they are heated and as a result they become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. Then the composite elements of the orthopaedic appliance are joined with the knitted fabric body and the ancillary elements such as fixing and welded straps. An adhesive is put between the body and the rigid composite elements. Following this operation, the product is sewn to finish and the stickers placed on the medium of the electric circuit to protect the active sites of the electric circuit acting on the patient's tissue are removed.

The elements of the orthopaedic appliance such as tension straps, surfaces with elements for bracing the product or other components of the end product are spot welded using a welding press. Then the two layers of the textile materials are bonded permanently after the adhesive between them melts under the temperature and the pressure force.

### Example V

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the knitted fabric,
- welding of the textile materials and composites,
- sewing to finish.

And the textile materials to be cut on the plotter are selected from among natural textile materials made of flax, jute, and cocoa or bamboo fibres.

The raw material is composed of technical fabrics made of natural fibres which contain up to 40% of PLA and/or PP fibres that are plasticised under temperature (90-200°C) and higher pressure, and as a polymer harden the other components of the fabric.

The composite is moulded with the use of a press, the outer layer of the composite is composed of the medium of the electric circuit/electronic system in the form of a grooved structure on the surface of the composite, which supports the electric circuit / electronic system.

The medium of the electric circuit / electronic system is put on a mould placed in the chamber and further layers of the composite are placed on the medium. Then the chamber is closed with a vacuum bag and sealed. After the pump is switched on, the infusion process is initiated which creates subpressure between the "vacuum bag" and the mould. At the same time liquid resin is pressed into the mould and it penetrates any free space in the textile material and permeates the material to the medium of electric circuit, thus bonding the medium together with the composite. The subpressure created in the vacuum chamber ranges from 0.3 to 0.9 bars.

The application of the vacuum method allows reducing the content of resin to as much as 50% compared to composites manufactured using traditional methods, with the same or even greater strength of the composite produced in this manner. During the infusion process subpressure is generated and as a result the applied fabrics are compressed with significant reduction of their volume and at the same time completely saturated with resin. Another important advantage of the application of the vacuum infusion technology is the elimination of air bubbles which increases the strength and aesthetic quality of the product.

When the composite is ready, the edges are processed so as to eliminate any technological allowances and also to make holes for screws or rivets. If the edges of the composite are too sharp, they are heated and as a result they become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. The body of the orthopaedic appliance is made of a knitted fabric thermostabilised to prevent shrinking of the knitted fabric elements of the end product in the use phase.

Then the composite elements of the orthopaedic appliance are joined with the knitted fabric body and the ancillary elements such as fixing and welded straps. The rigid composite elements are also placed in "pockets" or fixed with a Velcro strap or other tapes or bonded with an appropriate adhesive which has no destructive impact on the structure of the composite and the textile materials.

The elements of the orthopaedic appliance such as tension straps, surfaces with elements for bracing the product (including a composite insert with an electronic circuit) or other components of the end product are spot welded using a welding press. Then the two layers of the textile materials are bonded permanently after the adhesive between them melts under the temperature and the pressure force.

### Example VI

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the knitted fabric,
- welding of the textile materials and composites,
- sewing to finish.

The textile materials to be cut on the plotter are selected from among natural textile materials. The raw material is composed of technical fabrics made of natural fibres which contain up to 40% of PLA and/or PP fibres that are plasticised under temperature and higher pressure, and as a polymer harden the other components of the fabric.

The composite is moulded with the use of a RTM device and the outer layer of the composite is composed of a medium together with an electric circuit/electronic system. The medium of the electronic circuit/system is made as a grooved structure supporting the electric circuit/electronic system on the surface of the composite.

When the composite is moulded, the mould is first thoroughly cleaned and degreased and then coated with wax. The separator is then polished and a gel-coat is applied together with the medium with the electric circuit / electronic system. Then the layers of the textile material as cut out on the plotter are put into the mould. The moulds are sealed together and an adhesive is pressed inside them. Resin such as polyester resin, vinyl ester resin, epoxy resin or methacrylate resin is used as an adhesive.

The adhesive is continued to be pumped inside until it starts pouring through control outlets on the edges of the mould which means that the textile material is completely saturated in the total volume of the mould. Then the mould is opened and the ready-to-use product is taken out and the mould is prepared for the next cycle.

A great advantage of the RTM technology is the possibility of obtaining products with even and smooth surface on both sides which can be painted to class A, and also a relatively short duration of the production process. At the same time the resin transfer moulding allows that the thickness of the walls is precisely determined at each point of the adhesive.

When the composite is ready, the edges are processed so as to eliminate any technological allowances, and also to make holes for screws or rivets. If the edges of the composite are too sharp, they are heated and as a result become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. The body of the orthopaedic appliance is produced as a knitted fabric product. This knitted fabric is thermostabilised to prevent shrinking of the knitted fabric elements of the end product in the use phase.

Then the composite elements of the orthopaedic appliance are joined with the knitted fabric body and the ancillary elements such as fixing and welded straps. The rigid composite elements are placed in a "pocket" or fixed with a Velcro strap or tapes, or attached with an adhesive. Following this operation, the product is sewn to finish and the stickers placed on the medium of the electric circuit to protect its active sites acting on the patient's tissue are removed.

The elements of the orthopaedic appliance such as tension straps, surfaces with elements for bracing the product or other components of the end product are spot welded using a welding press. Then the two layers of the textile materials are bonded permanently after the adhesive between them melts under the temperature and the pressure force.

### Example VII

The method for producing orthopaedic appliances, specifically those provided with rigid components for active stimulation of the injured area, according to this invention is comprised of the following steps:
- cutting of the textile materials on a plotter,
- moulding of the composite,
- processing of the composite,
- finishing of the composite surface,
- producing of textiles for the body of the orthopaedic appliance,
- stabilising of the knitted fabric,
- welding of the textile materials and composites,
- sewing to finish.

And the textile materials to be cut on the plotter are selected from among natural textile materials made of flax, jute, and cocoa or bamboo fibres. The raw material is composed of technical fabrics made of natural fibres which contain up to 40% of PLA and/or PP fibres that are plasticised under temperature (90-200°C) and higher pressure, and as a polymer harden the other components of the fabric.

The composite is moulded with the use of a press, the outer layer of the composite is composed of the medium of the electric circuit/electronic system in the form of a grooved structure on the surface of the composite, which supports the electric circuit / electronic system.

When the composite is moulded, the medium together with the electric circuit is placed in the press mould, and then further layers of the composite are placed on the medium. The press is provided with a profile / mould of appropriate shape and heating elements, and has adjustable pressure force.

After all layers are placed, the press tool presses them into the mould and the layers of the fabric are combined under temperature and pressure into a uniform composite with the shape of the mould.

When the composite is ready, the edges are processed so as to eliminate any technological allowances and also to make holes for screws or rivets. If the edges of the composite are too sharp, they are heated and as a result become spontaneously rounded.

The composite with the surface prepared in this manner is varnished to protect it against mechanical damage and also for aesthetic reasons.

At the same time and regardless of the preparation of the composite elements, a knitted fabric is produced which is used for the body and the ancillary elements of the end product. Then the composite elements of the orthopaedic appliance are joined with the knitted fabric body and the ancillary elements such as fixing and welded straps. The rigid composite elements are placed in a "pocket" or fixed with a Velcro strap or tapes, or attached with an adhesive. Following this operation, the product is sewn to finish and the stickers placed on the medium of the electric circuit to protect its active sites acting on the patient's tissue are removed.

The elements of the orthopaedic appliance such as tension straps, surfaces with elements for bracing the product or other components of the end product are spot welded using a welding press. Then the two layers of the textile materials are bonded permanently after the adhesive between them melts under the temperature and the pressure force.

## Claims

1. The method for producing orthopaedic appliances, specifically those provided with rigid components for active stimulation of the injured area, according to this invention is comprised of the following steps:
• cutting of the textile materials on a plotter,
• moulding of the composite,
• processing of the composite,
• finishing of the composite surface,
• producing of textiles for the body of the orthopaedic appliance,
• stabilising of the knitted fabric,
• welding of the textile materials and composites,
• sewing to finish.
**characterised in that** the textile materials to be cut on the plotter are selected from among natural textile materials fortified with an admixture of fibres with low melting point, and the natural textile materials are made of flax, jute, coca or bamboo fibres doped with PP and/or PLA fibres, and these fibres contain up to 40% of PLA and/or PP fibres and are plasticised under the temperature of at least 90°C and up to a maximum of 200°C and pressure of 9 bars at maximum, and at least one external layer of the composite made of the natural textile materials fortified with PLA and/or PP fibres constitutes a carrier with at least one electric circuit / electronic system.

2. A method according to claim 1 **characterised in that** the carrier of the electric circuit/electronic system is a plastic which is plasticised under temperature and/or pressure, provided in the form of a film with an electronic circuit/system applied thereon with terminals protected with stickers.

3. A method according to claim 1 **characterised in that** the carrier of the electric circuit/electronic system is a grooved structure supporting the electric circuit /electronic system, made on the composite surface of textile materials fortified with PLA and/or PP fibres.

4. A method according to any of the claims referred to above **characterised in that** when the composite is moulded with the use of a press, the carrier with the electric circuit is placed in the mould, and then further layers of the composite are placed on the carrier, and the press is provided with a profile / mould with an appropriate shape, heating elements and has adjustable pressure force, and after all the layers are placed, the press tool presses them into the mould and the layers of the fabric are bonded under the temperature of at least 90°C up to a maximum of 200°C and pressure of 9 bars at maximum into a uniform composite with the shape of the mould.

5. A method according to any claim 1-3 **characterised in that** when the composite element is moulded in the vacuum chamber, the carrier of the electric circuit /electronic system is placed on the mould in the chamber, and then further layers of the composite on the carrier, and then the chamber is closed with a vacuum bag and sealed, and after the vacuum pump is switched on, liquid resin is pressed into the mould.

6. A method according to any claim 1-3 **characterised in that** when the composite element is moulded in the vacuum chamber, the mould in the chamber is shaped so as to enable producing a grooved structure of the carrier of the electric circuit, and after further layers of the composite are placed on the mould, the chamber is closed with a vacuum bag and sealed, and after a vacuum pump is switched on, the infusion process is initiated and as a result subpressure is created between the "vacuum bag" and the mould, and at the same time liquid resin is pressed into the mould and it penetrates any space of the textile material and permeates it to the carrier of the electric circuit, thus bonding it with the composite, and the subpressure created in the vacuum chamber ranges from 0.3 to 0.9 bars.

7. A method according to claims 1-3 **characterised in that** when the composite elements are moulded with the used of the RTM (Resin Transfer Moulding) method, first the mould is thoroughly cleaned and degreased, and then coated with a separator, which is polished, and then a gel-coat is applied thereon together with the carrier with the electric circuit / electronic system, and layers of the textile material are placed inside the mould, and the moulds are sealed together and an adhesive, such as resin from the group of polyester, vinyl ester, epoxy or methacrylate resins, is pressed into the moulds.

8. A method according to claim 7 **characterised in that** the adhesive is continued to be pumped inside until it starts pouring through control outlets on the edges of the mould.

9. A method according to any of the claims referred to above **characterised in that** when the composite is ready, the edges are processed so as to eliminate any technological allowances and also to make holes for screws or rivets, and if the edges of the composite are too sharp, they are heated and as a result become spontaneously rounded.

10. A method according to any of the claims referred to above **characterised in that** at the same time and regardless of the preparation of the composite elements, a knitted fabric is produced which is used for the body and the ancillary elements of the end product.

11. A method according to claim 10 **characterised in that** the body of the orthopaedic appliance is made of a knitted fabric, and this knitted fabric is thermostabilised.

12. A method according to any of the claims referred to above **characterised in that** at the same time and regardless of the preparation of the composite elements, the body of the orthopaedic appliance is made as an element sewn from ready-to-use textile materials.

13. A method according to any of the claims referred to above **characterised in that** the composite elements of the orthopaedic appliance are joined with a knitted-fabric body and ancillary elements such as fixing and welded straps, and an adhesive is put between the body and the rigid composite elements and/or the rigid composite elements are placed in a "pocket" or pockets, attached with Velcro straps or tapes.

14. A method according to claim 1 or 12 or 13 **characterised in that** after the composite element is put together with the body, the product is sewn to finish, and the elements of the orthopaedic appliance such as tension straps, surfaces with elements for bracing the product (including a composite insert with an electronic circuit) or other components of the end product are spot welded using a welding press.
